# EUROPÄISCHE PATENTSCHRIFT

(11) **EP 1 471 843 B1**
(45) Veröffentlichungstag und Bekanntmachung des Hinweises auf die Patenterteilung: **14.06.2006**
(21) Anmeldenummer: 02792802.7
(22) Anmeldetag: 26.11.2002
(51) Int. Cl.: A61B 17/72

(54) **EINRICHTUNG, INSBESONDERE MARKNAGEL UND/ODER HÜLSE ZUM EINSETZEN IN RÖHRENKNOCHEN**
DEVICE, ESPECIALLY AN INTRAMEDULLARY NAIL AND/OR A SLEEVE FOR INSERTING INTO TUBULAR BONES
DISPOSITIF, NOTAMMENT CLOU CENTRO-MEDULLAIRE ET/OU MANCHON A INSERER DANS DES OS LONGS

(30) Priorität: 06.02.2002 DE 10204904
(43) Veröffentlichungstag der Anmeldung: 03.11.2004
(73) Patentinhaber: Wittenstein AG, 97999 Igersheim (DE)
(72) Erfinder: KLEIN, Jürgen, 97999 Igersheim (DE); STAUCH, Roman, 97959 Assamstadt (DE)
(74) Vertreter: Weiss, Peter
(86) Internationale Anmeldenummer: PCT/EP2002/013276
(87) Internationale Veröffentlichungsnummer: WO 2003/065913

(56) Entgegenhaltungen:
- AT-B- 363 172
- DE-A- 19 813 914
- DE-U- 29 904 852
- US-A- 2 136 471
- US-A- 5 053 035
- US-B1- 6 197 029

## Beschreibung

Die vorliegende Erfindung betrifft eine Einrichtung, insbesondere Marknagel und/oder Hülse zum Einsetzen in Röhrenknochen aus einem Grundkörper, in welchem ggf. jeweils endseits radiale Bohrungen für Verriegelungsschrauben vorgesehen sind, wobei dem Grundkörper wenigstens ein Stabilisator zugeordnet ist.

Derartige Einrichtungen, insbesondere Marknägel sind in vielfältiger Form und Ausführung im Markt bekannt und erhältlich. Sie dienen im wesentlichen in der Chirurgie zum Verbinden zweier Segmente eines Röhrenknochens, zum Begradigen von Knochen, zum Distrahieren von Knochen ctc.. Dabei sollen auch unter dem Begriff des Marknagels sämtliche Distraktionsvorrichtungen fallen, die in einen Knochen eingeseLzL werden können um diesen zu verlängern.

Problematisch ist bei herkömmlichen Marknägeln, dass diese unzureichend in einen Knochen einzusetzen, und nur mit erheblichen Aufwand mit dem Knochen zu verbinden sind.

Dabei hat sich als nachteilig erwiesen, dass bei herkömmlichen in Röhrenknochen eingesetzte Marknägel ein gewisses Spiel auftritt und hierdurch die Präzision bspw. beim Begradigen des Knochens erheblich darunter leidet.

Durch dieses Spiel lässt sich bspw. in radialer Richtung der getrennte Knochen sich bewegen, was unerwünscht ist und zu keinem präzisen Zusammenwachsen der Knochensegmente führt.

Die AT 363 172 B offenbart einen Markhöhlennagel, der eine Mehrzahl von stufenartig abgesetzten Lamellen radial aufweist. Zwischen den einzelnen Lamellenabschnitten sind entsprechende Schneidkanten gebildet. Der Oberbegriff des Anspruchs 1 basiert dieser Offenbarung.

Die US 6,197,029 B1 offenbart einen Marknagel, der einends Stabilisatorflossen aufweist, deren Aussenkontur im wesentlichen parallel zur Oberfläche des Marknabels verlaufen. Einen ähnlichen Marknagel zeigte die US 2,136,471 A sowie die DE 198 13 914 A1 und die US 5,053,035 sowie DE 299 04 852 U1. Bei all diesen medizinischen Marknägeln sind die Aussenkonturen, deren Stabilisatoren im wesentlichen parallel verlaufend über die gesamte Länge des Marknagels zur Oberfläche des Marknagels bzw. deren Hülse ausgerichtet.

Der vorliegenden Erfindung liegt daher die Aufgabe zugrunde eine Einrichtung, insbesondere Marknagel oder Hülse der eingangs genannten Art zu schaffen, mit welcher auf kostengünstige effektive und präzise Weise ein Einsetzen in einen Röhrenknochen erleichtert, ein Festlegen optimiert und ein Spiel im Röhrenknochen minimiert ist.

Zur Lösung dieser Aufgabe führt, dass der zumindest eine Stabilisator zumindest eine Aufnalunebohrung rür Verricgelungsschrauben aufweist.

Bei der vorliegenden Erfindung sind vorzugsweise entweder am proximalen oder distalen Ende der Einrichtung, insbesondere des Marknagels oder der Hülse in Axialrichtung eine Mehrzahl von vorzugsweise lotrecht von einer Grundkörperoberfläche des Marknagels oder der Hülse abragende Stabilisatoren vorgesehen, die vorzugsweise gegenüberliegend sternförmig oder in drehbaren Winkeln zueinander angeordnet sind.

Diese sind bspw. keilartig ausgebildet und verlaufen in Längsrichtung von einer Erhöhung in eine Spitze, die dem gegenüberliegenden Ende des Marknagels ausgerichtet ist.

Querschnittlich sind die Stabilisatoren vorzugsweise dreieckartig ausgebildet und ggf. mit einer scharfen Aussenkante versehen.

Wird bspw. die Einrichtung, insbesondere der Marknagel oder die Hülse proximal oder distal in einen Röhrenknochen eingeführt, so durchgreift das eine Ende den Röhrenknochen stirnseitig und das andere Ende lässt sich am distalen oder proximalen Ende in den Röhrenknochen einschlagen, so dass sich die Stabilisatoren, insbesondere mit ihrer scharfen Aussenkante in eine Innenwand des Röhrenknochens einschneiden. Hierdurch wird eine optimale Zentrierung des Marknagels oder der Hülse im Röhrenknochen gewährleistet und gleichzeitig eine Verdrehsicherung im Röhrenknochen realisiert.

Hierdurch lässt sich sehr präzise und exakt der Marknagel im Röhrenknochen rotationsfest einsetzen und eignet sich daher besonders für Korrekturen der Rotation des Röhrenknochens. Zudem wird durch das Eingreifen der scharfen Aussenkante und durch das Eingreifen in den weichen Knochenanteil innen am distalen oder proximalen Ende aller Röhrenknochen die Festigkeit des Marknagels bzw. des Implantates erheblich erhöht. Dabei kann der Marknagel von jeder beliebigen Seite antegrad oder retrograd in den Röhrenknochen implantiert werden.

Ferner ist von Vorteil, dass der Stabilisator entsprechende Aufnahmebohrungen aufweist, die ausserhalb einer Mittelachse liegen, so dass durch diese externen Aufnahmebohrungen eine äusserst gute Festlegung und Verschraubung mit dem Röhrenknochen erfolgen kann. Die Verschraubung ist daher lediglich im äusseren Mantelbereich des Röhrenknochens vorzusehen, diese muss nicht mehr den Röhrenknochen vollständig durchgreifen. Dies ist ebenfalls ein weiterer Vorteil. Insbesondere durch die extreme Rotationsfestigkeit des Marknagels im Röhrenknochen hat sich dieser besonders ausgezeichnet.

Ist die Einrichtung bspw. als Hülse mit den o. g. Stabilisatoren ausgebildet, so lässt sich am proximalen oder distalen Ende stirnseitig in den Knochen diese Hülse in den Markraum bspw. eines Röhrenknochens eintreiben. Über die entsprechenden Stabilisatoren wird die Hülse innerhalb des Knochens gehalten und lässt sich in beschriebener Weise mittels Befestigungselementen dort festlegen.

Dann kann in diese Hülse ein herkömmlicher, beliebiger Marknagel eingeführt werden, wird dort fixiert und festgehalten. Eine entsprechende Verrieglungseinrichtung legt den Marknagel ggf. vorgespannt innerhalb der Hülse fest. Hierdurch können bspw. Marknägel mit sensiblen Distraktionsvorrichtungen etc. sehr schonend und wiederverwendbar in eine entsprechende Hülse eingeführt, fixiert und mit dieser verriegelt werden. Bevorzugt ist stirnseitig einends der Hülse eine Verriegelung bspw. eine Mutter od. dgl. vorgesehen, wobei zwischen der Verriegelung und dem eingesetzten Marknagel bspw. ein Kraftspeicherelement, insbesondere ein Federelement zum Vorspannen bzw. zur Dynamisierung, insbesondere zur Förderung des Knochenwachstums des eingesetzen Marknagels vorgesehen sein kann.

Auch soll daran gedacht sein, den Marknagel radial verdrehsicher in die Bohrung der Hülse einzuschieben. Dies kann über ggf. Rastnuten, keilwellenartige Rastnasen od. dgl. geschehen. Auch ist denkbar, bspw. auch andere Querschnitte, ovale Querschnitte von Marknagel und Bohrung der Hülse zu verwenden und um ein axiales Einführen zu gewährleisten und ein radiales Verdrehen zu verhindern.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine schematisch dargestellte Draufsicht auf einen erfindungsgemässen Marknagel mit Stabilisatoren;
Figur 2 einen schematisch dargestellten Querschnitt durch den Marknagel gemäss Figur 1 entlang Linie II-II;
Figur 3 eine schematisch dargestellte Draufsicht auf ein weiteres Ausführungsbeispiel eines weiteren Marknagels gemäss Figur 1;
Figur 4 einen schematisch dargestellten Querschnitt durch den Marknagel gemäss Figur 3 entlang Linie IV-IV;
Figuren 5a und 5b zeigen weitere Ausführungsbeispiele, weiterer Querschnitte durch einen Marknagel gemäss den Figuren 1 und 3;
Figur 6 eine schematisch dargestellte vergrösserte Draufsicht eines möglichen Stabilisators;
Figur 7 einen schematisch dargestellten Querschnitt durch eine Hülse zum Eintreiben in einen Markraum eines Knochens;
Figur 8 eine schematisch dargestellte Seitenansicht auf die Hülse gem. Figur 7 mit eingesetztem Marknagel entlang Linie VIII-VIII.

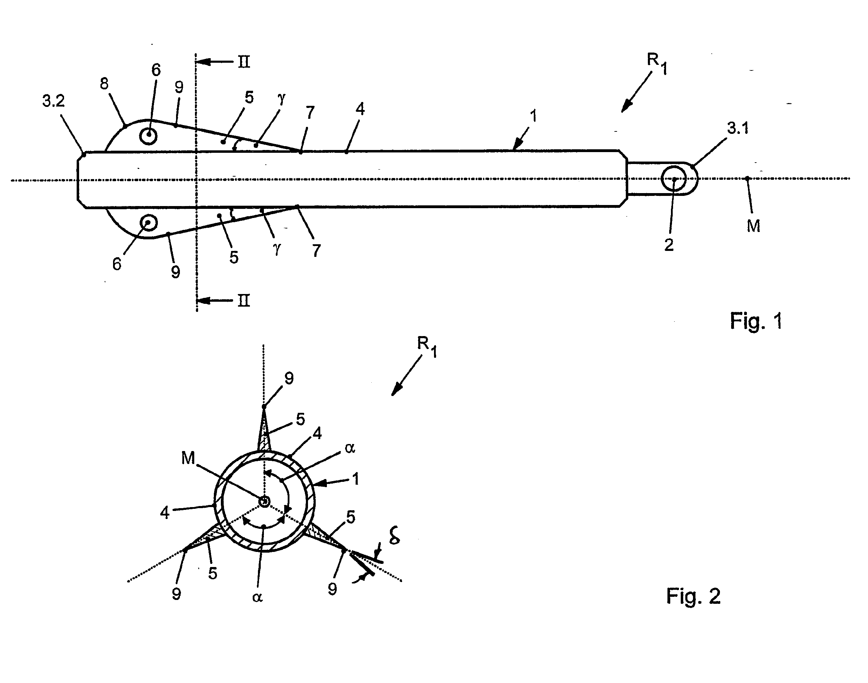

Gemäss Figur 1 weist eine erfindungsgemässe Einrichtung, insbesondere Marknagel R₁ einen Grundkörper 1 auf, welcher querschnittlich, wie es in Figur 2 dargestellt ist, röhrenartig ausgebildet sein kann. Der Marknagel R₁ kann an einem Ende 3.1 mit einer Bohrung 2 versehen sein, um hier nicht näher dargestellte Verriegelungsschrauben od. dgl. durchzuführen, um den Marknagel R₁ an einem hier nicht dargestellten Knochen festzulegen.

Der Marknagel R₁ kann als röhrenartiger Grundkörper 1 ausgebildet sein. Er kann auch aus Vollmaterial, Verbundmaterial, in beliebigen Querschnitten hergestellt sein.

Auch soll im Rahmen der vorliegenden Erfindung liegen den Marknagel R₁ als Distraktionsvorrichtung mit zwei gegeneinander bewegbaren Elementen zum Distrahieren von Knochen auszubilden. Hierauf sei die vorliegende Erfindung nicht beschränkt.

Vorzugsweise ist der Grundkörper 1 um eine Mittelachse M rotationssymetrisch in Längsrichtung ausgebildet und weist an seiner Grundkörperoberfläche 4 eine Mehrzahl von Stabilisatoren 5 auf. Die Stabilisatoren 5 enthalten Aufnahmebohrungen 6, die ebenfalls wie auch die Bohrung 2 zum Aufnehmen von Verriegelungsschrauben dienen.

Es ist bei der vorliegenden Erfindung vorzuziehen, dass die Stabilisatoren , 5 flossenartig in Längsrichtung des Grundkörpers 1 von der Grundkörperoberfläche 4 abragen und eine Spitze 7 des Stabilisators 5 gegen das Ende 3.1 des Grundkörpers 1 ausgerichtet ist. Von der Spitze 7 verläuft in einem spitzen Winkel γ der Stabilisator 5 zu einem zweiten Ende 3.2 des Grundkörpers 1 in eine Erhöhung 8 über und gelangt dort vorzugsweise abgerundet zur Grundkörperoberfläche 4. Im Bereich der Erhöhung 8 ist vorzugsweise die Aufnahmebohrung 6 vorgesehen.

Querschnittlich betrachtet ist der Stabilisator, wie es insbesondere aus Figur 2 hervorgeht, dreieckartig ausgebildet und weist eine scharfe Aussenkante 9 auf. Vorzugsweise sind mehrere Stabilisatoren 5 auf der Grundkörperoberfläche 4, wie es in den Figuren 1 und 2 dargestellt ist angeordnet.

Bei dem Marknagel R₁ sind vorzugsweise im Winkel α = 120° zur Mittelachse M drei Stabilisatoren 5 beabstandet angeordnet. Dies hat den Vorteil, dass beim Einsetzen, insbesondere beim Einschlagen des Marknagels R₁ in einen Röhrenknochen sich im Bereich des Endes 3.2 der Marknagel R₁ sich im Knochen zentriert und sich die Aussenkanten 9 innen in den Röhrenknochen einschneiden. Hierdurch wird eine Rotationssicherung des Marknagels R₁ im Knochen gewährleistet.

Ferner ist vorteilhaft, dass aussermittig, insbesondere exzentrisch über die Aufnahmebohrungen 6 Verriegelungsschrauben eingesetzt werden können, um den Marknagel R₁ gegenüber dem Röhrenknochen festzulegen.

Vorzugsweise bilden die Stabilisatoren 5 querschnittlich einen spitzen Winkel δ aus, wie es in Figur 2 dargestellt ist.

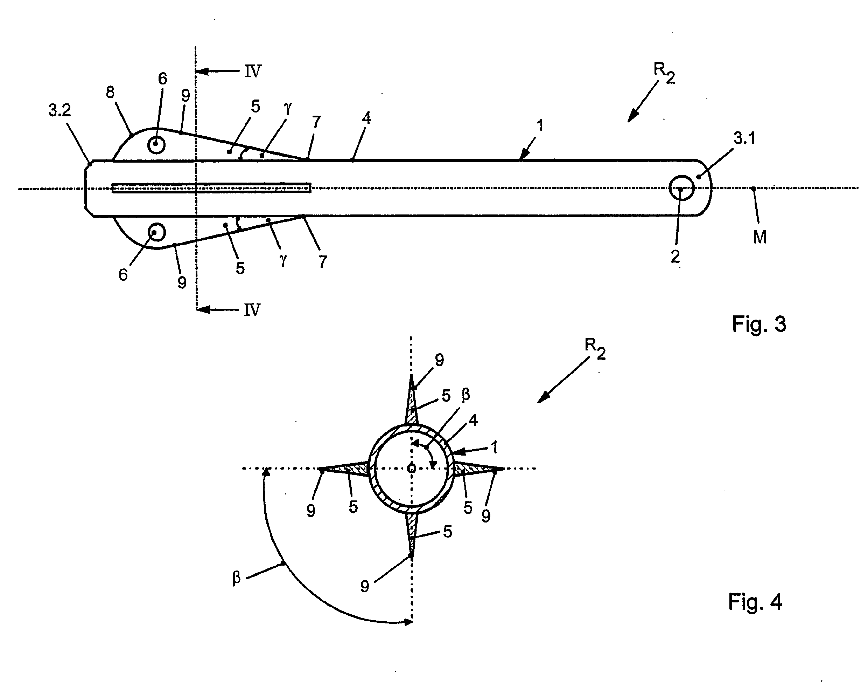

In dem Ausführungsbeispiel der Figuren 3 und 4 ist ein Marknagel R₂ dargestellt, wobei die Stabilisatoren 5 auch in Längsrichtung axial vorzugsweise im Bereich des Endes 3.2 des Grundkörpers 1 angeordnet sind. Dabei sind die Stabilisatoren 5 von der Spitze 7 zum Ende 3.2 hin verlaufend im spitzen Winkel γ keilartig gebildet, wobei im Bereich einer Erhöhung 8 die Aufnahmebohrung 6 vorgesehen ist.

Unterschiedlich ist bei dem Marknagel R₂ gemäss den Figuren 3 und 4, dass die einzelnen Stabilisatoren 5 jeweils zueinander etwa im Winkel β = 90° auf der Grundkörperoberfläche 4 angeordnet sind.

Die einzelnen Stabilisatoren 5 können fest verschweisst, hart gelötet oder mit dem Grundkörper 1 bspw. verklebt oder sogar vernietet sein. Dies soll ebenfalls im Rahmen der vorliegenden Erfindung liegen.

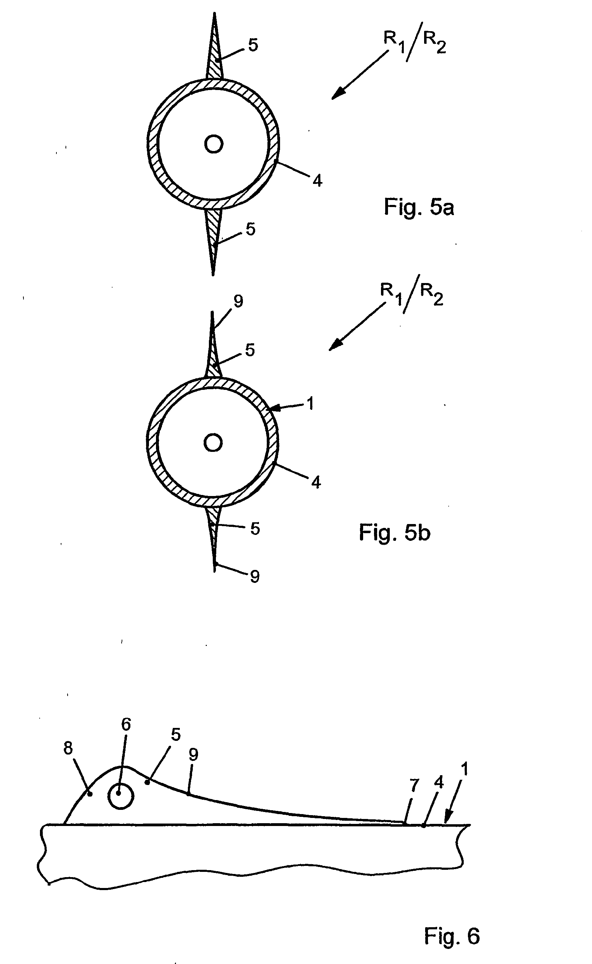

Wie es ferner in den Figuren 5a und 5b dargestellt ist, können die einzelnen Stabilisatoren 5 eines beliebigen Marknagels R₁/R₂ dreieckartig oder leicht nach innen gewölbt, wie es in Figur 5b dargestellt ist ausgebildet sein. Hierdurch lässt sich Einfluss auf eine Dicke des Stabilisators 5 insbesondere im Bereich der scharfen Aussenkanten 9 nehmen. Je dünner die Aussenkante 9 ausgebildet ist, je weiter lässt sie sich in den Knochen in dessen Innenwand einschlagen.

Auch soll daran gedacht, dass die Aussenkante 9, wie es insbesondere in Figur 6 dargestellt ist, eines beliebigen Stabilisators 5 kurvenartig gewölbt zur Grundkörperoberfläche 4 ausgebildet sein kann. Dies erleichtert unter Umständen ein Einschneiden der Aussenkante 9 innen in den Röhrenknochen. Vorzugsweise ist die Spitze 7 gegen das Ende 3.1 siehe Figur 1 ausgerichtet, so dass beim Einschlagen des Marknagel R₁ in den Röhrenknochen im distalen oder proximalen Ende eine Verriegelung für alle beliebigen Röhrenknochen mit optimierter Rotationssicherung gewährleistet wird. Durch die externe ausserhalb der Mittelachse M liegende Aufnahmebohrung 6 im Stabilisator 5 lassen sich auch divergierende oder konvergierende Schraubenanordnungen zur Festlegung des Marknagels im Röhrenknochen realisieren.

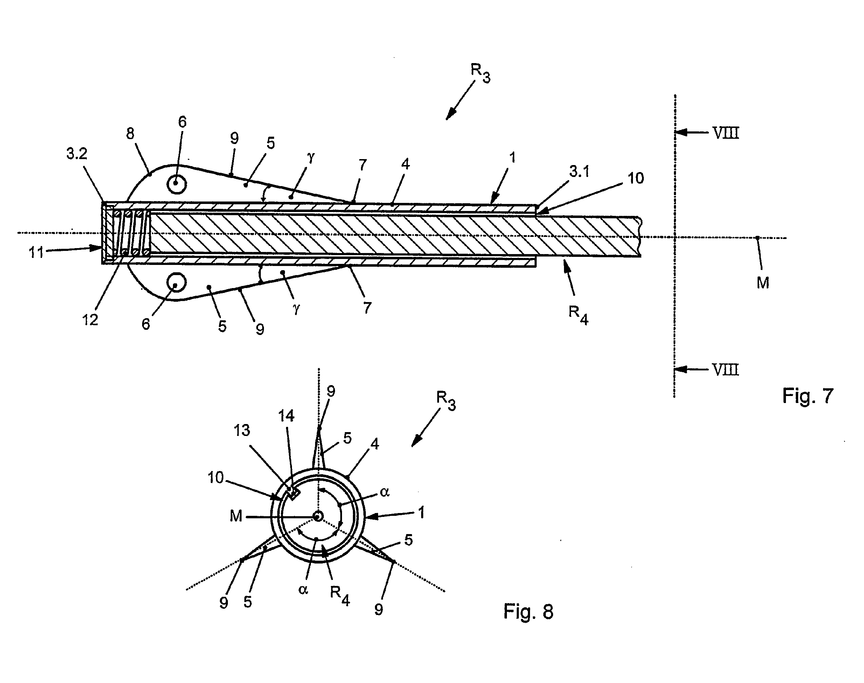

In einem weiteren Ausführungsbeispiel der vorliegenden Erfindung gemäss den Figuren 7 und 8 ist eine Einrichtung als Hülse R₃ ausgebildet, die in etwa der oben beschriebenen Art entspricht. Unterschiedlich ist, dass die Hülse R₃ als hülsenartiges Element ausgebildet ist und mit einer Bohrung 10 als Durchgangsbohrung versehen ist. An der Grundkörperoberfläche 4 sind wie oben beschrieben, die Stabilisatoren 5 angeordnet. Unterschiedlich ist hier, dass eine derartige Hülse R₃ sehr leicht bspw. vom proximalen oder distalen Ende eines Knochen aus in die Markraumhöhle des Knochens eingetrieben werden kann. Diese sitzt dann dort fixiert und lässt sich über die Aufnahmebohrungen 6 mit dem Knochen in oben beschriebener Weise festlegen.

Durch die Bohrung 10 kann dann ein herkömmlicher Marknagel R₄, wie er insbesondere in Figur 7 dargestellt ist, eingesetzt werden.

Dabei können Marknägel R₄ auch als Distraktionsvorrichtungen eingesetzt werden, die empfindliche Bauteile aufweisen.

Endseits ist vorzugsweise im Bereich des Endes 3.2 des Grundkörpers 1 der Hülse R₃ eine Verriegelungseinrichtung 11 vorgesehen, um den eingesetzten Marknagel R₄ gegen axiales Verschieben zu sichern. Ggf. kann zwischen Verriegelungseinrichtung 11 und Marknagel R₄ ein Kraftspeicherelement 12 eingesetzt sein, um eine entsprechende axiale Vorspannung des Marknagel R₄ zu erzeugen. Als Verriegelungseinrichtung 11 kann bspw. eine Schraube, eine Mutter od. dgl. Einrichtung dienen. Hierauf sei die vorliegende Erfindung nicht beschränkt.

Ferner soll daran gedacht sein, wie es insbesondere in Figur 8 angedeutet ist, den Marknagel R₄ in die Hülse R₃ gegen radiales Verdrehen um die Mittelachse M zu sichern, indem bspw. ein Rastelement 13 innen vom Grundkörper 1 abragt und in eine entsprechende fluchtend passende Ausnehmung 14 des Marknagels R₄ eingreift, so dass lediglich der Marknagel R₄ axial gegenüber der Hülse R₃ entlang der Mittelachse M hin und her bewegbar ist. Dabei können auch mehrere entsprechende Rastelement 13 und Ausnehmungen 14 vorgesehen sein.

Die Hülse R3 ermöglicht ein einfaches Austauschen, Fixieren, Zentrieren, Einsetzen oder Entnehmen eines beliebigen Marknagels, wobei gleichzeitig die Hülse R₃ als Gewebeschutz dient.

Zudem wird realisiert, dass bspw. der Marknagel R₄ axial vorgespannt in die Hülse R₃ bzw. in den Knochen eingeführt werden kann.

### Positionszahlenliste

| | | | | | |
|---|---|---|---|---|---|
| 1 | Grundkörper | 34 | | 67 | |
| 2 | Bohrung | 35 | | 68 | |
| 3 | Ende | 36 | | 69 | |
| 4 | Grundkörperoberfläche | 37 | | 70 | |
| 5 | Stabilisator | 38 | | 71 | |
| 6 | Aufnahmebohrung | 39 | | 72 | |
| 7 | Spitze | 40 | | 73 | |
| 8 | Erhöhung | 41 | | 74 | |
| 9 | Aussenkante | 42 | | 75 | |
| 10 | Bohrung | 43 | | 76 | |
| 11 | verriegelungs-Einrichtung | 44 | | 77 | |
| 12 | Kraftspeicherelement | 45 | | 78 | |
| 13 | Rastelement | 46 | | 79 | |
| 14 | Ausnehmung | 47 | | | |
| 15 | | 48 | | | |
| 16 | | 49 | | R₁ | Marknagel |
| 17 | | 50 | | R₂ | Marknagel |
| 18 | | 51 | | R₃ | Hülse |
| 19 | | 52 | | R₄ | Marknagel |
| 20 | | 53 | | | |
| 21 | | 54 | | | |
| 22 | | 55 | | | |
| 23 | | 56 | | M | Mittelachse |
| 24 | | 57 | | | |
| 25 | | 58 | | | |
| 26 | | 59 | | | |
| 27 | | 60 | | α | Winkel |
| 28 | | 61 | | β | Winkel |
| 29 | | 62 | | γ | Winkel |
| 30 | | 63 | | | |
| 31 | | 64 | | | |
| 32 | | 65 | | | |
| 33 | | 66 | | | |

## Patentansprüche

1. Einrichtung, insbesondere Marknagcl (R₁, R₂) und/oder Hülse (R₃) zum Einsetzen in Röhrenknochen, aus einem Grundkörper (1), in welchem ggf. jeweils endscits radiale Bohrungen (2) für Verriegelungsschrauben vorgesehen sind, wobei dem Grundkörper (1) wenigstens ein in Axialrichtung des Grundkörpers (1) angeordneter und von diesen abragender Stabilisator (5) zugeordnet ist,
**dadurch gekennzeichnet,**
**dass** der zumindest eine Stabilisator (5) zumindest eine Aufnahmebohrung (6) für Verriegelungsschrauben aufweist,

2. Einrichtung nach Anspruch 1, **dadurch gekennzeichnet, dass** der zumindest eine Stabilisator (b) keilartig ausgebildet ist, wobei eine Spitze (7) des Stabilisators (5) gegen ein gegenüberliegendes Ende (3.1 oder 3.2) des Grundkörpers (1) in Axialrichtung ausgerichtet ist und die Aussenkante (9) des Stabilisators (5) zwischen Spitze (7) und Erhöhung (8) geradlinig oder gewölbt ausgebildet und zur der Grundkörperoberfläche (4) einen spitzen Winkel (γ) einschliesst.

3. Einrichtung nach Anspruch 1 oder 2, **dadurch gekennzeichnet, dass** der zumindest eine Stabilisator (5) cinends dem Grundkörper (1) zugeordnet isL und von einer Grundkörperoberflache (4) abragt.

4. Einrichtung nach wenigstens einem der Ansprüche 1 bis 3, **dadurch** qekennzeichnet, dass eine Mehrzahl von Stabilisaloreu (5) zumindest einends radial um den Grundkörper (1) angeordnet sind und jeweils in Axialrichtung ausgerichtet sind.

5. Einrichtung nach wenigstens einem der Ansprüche 1 bis 4, **dadurch gekennzeichnet, dass**, die Stabilisatoren (5) keilförmig ausgebildet sind.

6. Einrichtung nach wenigstens einem der Ansprüche 2 bis 5, **dadurch gekennzeichnet, dass** die Stabilisatoren (5) in etwa lotrecht von der Grundkörperfläche (4) des Grundkörpers (1) ausgerichtet und in Axialrichtung abragen.

7. Einrichtung nach wenigstens einem der Ansprüche 1 bis 6, **dadurch gekennzeichnet, dass** der zumindest eine Stabilisator (5) eine scharfe Aussenkannte (9) aufweist.

8. Einrichtung nach,wenigstens einem der Ansprüche 1 bis 7, **dadurch gekennzeichnet, dass** der stabilisator (5) querschnittlich dreieckartig ausgebildet ist.

9. Einrichtung nach wenigstens einem der Ansprüche 1 bis 8, **dadurch gekennzeichnet, dass** zwei Stabilisatoren (5) jeweils gegenüberliegend endscits dem Grundkörper (1) zugeordnet sind.

10. Einrichtung nach wenigstens einem der Ansprüche 1 bis 9, **dadurch gekennzeichnet, dass** drei Stabilisatoren (5) radial 1 endseits um den Grundkörper (1) im Winkel α von jeweils 120° zueinander angeordnet sind.

11. Einrichtung nach wenigstens einem der Ansprüche 1 bis 10, **dadurch gekennzeichnet, dass** die Stabilisatoren (5) radial um den Grundkörper im Winkel β = 90° zueinander beabstandet angeordnet sind.

12. Einrichtung nach wenigstens einem der Ansprüche 1 bis 11, **dadurch gekennzeichnet, dass** der Stabilisator (5) einends eine Erhöhung (8) aufweist, in dessen Bereich die Aufnahmebohrung (6) vorgesehen ist.

13. Einrichtung nach wenigstens einem der Ansprüche 1 bis 12, **dadurch gekennzeichnet, dass** der zumindest eine Stabilisator (5) mit seiner Spitze (7) in einer Einschlagrichtung des Grundkörpers (1) in einen Röhrenknochen axial ausgerichtet ist.

14. Einrichtung nach wenigstens einem der Ansprüche 1 bis 13, **dadurch gekennzeichnet, dass** die Hülse (R₃) eine Bohrung (10) zum Einstecken eines Marknagels (R₄) aufweist.

15. Einrichtung nach wenigstens einem der Ansprüche 1 bis 14, **dadurch** qekennzeichnet, dass ein beliebiger Marknagel (R₁) in der Hülse (R₃) fixierbar, insbesondere radial verdrehfest einschiebbar ist.

16. Einrichtung nach wenigstens einem der Ansprüche 1 bis 15, **dadurch gekennzeichnet, dass** In die Hülse (R₃) ein beliebiger Marknagel (Rg) axial einschiebbar und gegenüber der Hülse (R₃) mittels zumindest einer Verriegelungseinrichtung (11) festlegbar ist.

17. Einrichtung nach Anspruch 16, **dadurch gekennzeichnet, dass** der Marknagel (R₄) axial vorspannbar in die Hülse (R₃) einsetzbar ist, wobei die verriegelungseinrichtung (11) cinends im Bereich des Endes (3.2) vorgesehen ist.

18. Einrichtung nach Anspruch 16 oder 17, **dadurch gekennzeichnet, dass** einends des Marknagels (R₄) und der Verrieqelungseüirichtung (11) der Hülse (R₃) zum axialen vorspannen zumindest ein Kraf tspeictierelcment (12), insbesondere Federelment vorgesehen ist.

19. Einrichtung nach wenigstens einem der Ansprüche 14 bis 18, **dadurch gekennzeichnet, dass** der Marknagel (R₄) in der Bohrung (10) der Hülse (R₃) radial verdrehsicher und axial verschiebbar in diese einsetzbar ist, wobei ggf. Rastelemente (13) und Ausnehmungen (14) von Hülse (R₃) und/oder Marknagel (R₄), die radiale Verdrehsicherung bilden.

## Claims

1. Device, more especially an intramedullary nail (R₁, R₂) and/or a sleeve (R₃), for inserting into tubular bones, said device being formed from a basic body (1), in which are provided, possibly at each end, radial bores (2) for locking screws, at least one stabiliser (5), disposed in the axial direction of the basic body (1) and protruding therefrom, being associated with the basic body (1), **characterised in that** the at least one stabiliser (5) has at least one receiving bore (6) for locking screws.

2. Device according to claim 1, **characterised in that** the at least one stabiliser (5) has a wedge-shaped configuration, one tip (7) of the stabiliser (5) being orientated in the axial direction relative to an oppositely situated end (3.1 or 3.2) of the basic body (1), and the outer edge (9) of the stabiliser (5) between tip (7) and raised portion (8) having a rectilinear or curved configuration and forming an acute angle (γ) with the basic body surface (4).

3. Device according to claim 1 or 2, **characterised in that** the at least one stabiliser (5) is associated with one end of the basic body (1) and protrudes from a basic body surface (4).

4. Device according to at least one of claims 1 to 3, **characterised in that** a plurality of stabilisers (5) are disposed at least at one end radially about the basic body (1) and are orientated respectively in the axial direction.

5. Device according to at least one of claims 1 to 4, **characterised in that** the stabilisers (5) have a wedge-shaped configuration.

6. Device according to at least one of claims 2 to 5, **characterised in that** the stabilisers (5) are orientated substantially vertically from the basic body surface (4) of the basic body (1) and protrude in the axial direction.

7. Device according to at least one of claims 1 to 6, **characterised in that** the at least one stabiliser (5) has a sharp outer edge (9).

8. Device according to at least one of claims 1 to 7, **characterised in that** the stabiliser (5) has a triangular cross-section.

9. Device according to at least one of claims 1 to 8, **characterised in that** two stabilisers (5) are associated with the basic body (1) at respectively oppositely situated ends.

10. Device according to at least one of claims 1 to 9, **characterised in that** three stabilisers (5) are disposed radially at one end about the basic body (1) at an angle α of respectively 120° relative to one another.

11. Device according to at least one of claims 1 to 10, **characterised in that** the stabilisers (5) are disposed radially about the basic body, spaced apart from one another at an angle β = 90°.

12. Device according to at least one of claims 1 to 11, **characterised in that** the stabiliser (5) has a raised portion (8) at one end, in the region of which the receiving bore (6) is provided.

13. Device according to at least one of claims 1 to 12, **characterised in that** the at least one stabiliser (5) is axially orientated with its tip (7) in a direction in which the basic body (1) is driven into a tubular bone.

14. Device according to at least one of claims 1 to 13, **characterised in that** the sleeve (R₃) has a bore (10) for the insertion of an intramedullary nail (R₄).

15. Device according to at least one of claims 1 to 14, **characterised in that** any desirable intramedullary nail (R₄) can be secured in the sleeve (R₃), more especially it can be pushed-in in a radially non-rotatable manner.

16. Device according to at least one of claims 1 to 15, **characterised in that** any desirable intramedullary nail (R₄) can be axially pushed into the sleeve (R₃) and can be secured relative to the sleeve (R₃) by means of at least one locking device (11).

17. Device according to claim 16, **characterised in that** the intramedullary nail (R₄) is insertable into the sleeve (R₃) in an axially initially tensionable manner, the locking device (11) being provided at one end in the region of the end (3.2).

18. Device according to claim 16 or 17, **characterised in that** at least one force storage element (12), more especially a resilient element, is provided at one end of the intramedullary nail (R₄) and of the locking device (11) of the sleeve (R₃) for the axial initial tensioning.

19. Device according to at least one of claims 14 to 18, **characterised in that** the intramedullary nail (R₄) in the bore (10) of the sleeve (R₃) is insertable therein in a radially twist-preventing and axially displaceable manner, locking elements (13) and recesses (14) of the sleeve (R₃) and/or of the intramedullary nail (R₄) possibly forming the radial twist-preventing means.

## Revendications

1. Dispositif, en particulier clou centro-médullaire (R₁, R₂) et/ou manchon (R₃), à insérer dans des os longs, composé d'un corps de base (1) dans lequel sont éventuellement prévus à chaque extrémité des alésages radiaux (2) pour des vis de verrouillage, au corps de base (1) étant associé au moins un stabilisateur disposé dans le sens axial du corps de base (2) et faisant saillie par rapport à ce dernier,
**caractérisé par le fait que**
l'au moins un stabilisateur (5) présente au moins un alésage de réception (6) de vis de verrouillage.

2. Dispositif selon la revendication 1, **caractérisé par le fait que** l'au moins un stabilisateur (5) est réalisé sous forme de coin, une pointe (7) du stabilisateur (5) étant orientée dans, le sens axial par rapport à une extrémité opposée (3.1 ou 3.2) du corps,de base (1) et le bord extérieur (9) du stabilisateur (5) étant réalisé, entre la pointe (7) et le rehaussement (8), de manière linéaire ou bombé et forme, avec la surface du corps de base (4), un angle aigù (γ).

3. Dispositif selon là revendication 1 ou 2, **caractérisé par le fait que** l'au moins un stabilisateur (5) est associe, a une extrémité, au corps de base (1) et fait saillie par rapport à une surface de corps de base (4).

4. Dispositif selon au moins l'une des revendications 1 à 3, **caractérisé par le fait qu'**une pluralité de stabilisateurs (5) sont disposés, du moins, à une extrémité, radialement autour du corps de base (1) et sont orientés; chacun, dans le sens axial.

5. Dispositif selon au moins l'une des revendications ,1 à 4, **caractérisé par le fait que** les stabilisateurs (5) sont réalisés en forme de coin.

6. Dispositif selon au,moins 1,'une des revendications 2 à 5, **caractérisé par le fait que** les stabilisateurs (5) font saillie de manière orientée environ verticalement par rapport à la surface de corps de basé (4) du corps de base (1) et dans le sens axial.

7. Dispositif selon au moins l'une des revendications 1 à 6, **caractérisé par le fait que** l'au moins un stabilisateur (5) présente un bord extérieur tranchant (9).

8. Dispositif selon au moins l'une des revendications 1 à 7, **caractérisé par le fait que** le stabilisateur (5) est réalisé de section triangulaire.

9. Dispositif selon au moins l'une des revendications 1 à 8, **caractérisé par le fait que** deux stabilisateurs (5) sont associés au corps de base (1), du côté de l'extrémité, de manière opposée l'un à l'autre.

10. Dispositif selon au moins l'une des revendications 1 à 9, **caractérisé par le fait que** trois stabilisateurs (5) sont disposés du côté de l'extrémités, radialement autour du corps de base (1), selon un angle α de chaque fois 120° entre eux.

11. Dispositif selon au moins l'une des revendications 1 à 10, **caractérisé par le fait que** les stabilisateurs (5) sont disposés radialement autour du corps de base, distants d'un angle β = 90° entre eux.

12. Dispositif selon au moins l'une des revendications 1 à 11, **caractérisé par le fait que** le stabilisateur, (5) présente, à l'extrémité, un rehaussement (8) à l'endroit duquel est prévu l'alésage de réception (6).

13. Dispositif selon au moins l'une des revendications 1 à 12, **caractérisé par le fait que** l'au moins un stabilisateur (5) est orienté axialement avec sa pointe (7) dans une direction d'insertion du corps de base (1) dans un os long.

14. Dispositif selon au moins l'une des revendications 1 à 13, **caractérisé par le fait que** le manchon (R₃) présente un alésage (10) pour l'insertion d'un clou centro-médullaire (R₄).

15. Dispositif selon au moins l'une des revendications 1 à 14, **caractérisé par le fait qu'**un clou centre-médullaire (R₄) quelconque peut être fixé dans le manchon (R₃), en particulier inséré de manière fixe en rotation radiale.

16. Dispositif selon au moins l'une des revendications 1 bis 15, **caractérisé par le fait qu'**un clou centro-médullaire (R₄) peut être inséré axialement dans le manchon (R₃) et être fixé par rapport au manchon (R₃) au moyen d'au moins un dispositif de verrouillage (11);

17. Dispositif selon la revendication 16, **caractérisé par le fait que** le clou centro-médullaire (R₄) peut être inséré de manière prétendu axialement dans le manchon (R₃), le dispositif de verrouillage (11) étant prévu à une extrémité à l'endroit de l'extrémité (3.2).

18. Dispositif selon la revendication 16 ou 17, **caractérisé par le fait qu'**à une extrémité du clou centro-médullaire (R₄) et du dispositif de verrouillage (11) du manchon (R₃) est prévu, en vue de la prétension axiale, au moins un élément d'accumulation de force (12), en particulier un élément de ressort.

19. Dispositif selon au moins l'une des revendications 14 à 18, **caractérisé par le fait que** le clou centro-médullaire (R₄) peut être inséré dans l'alésage (10) du manchon (R₃) de manière fixe en rotation radiale et mobile axialement dans ce dernier, des éléments d'encliquetage (13) et évidements (14) du manchon (R₃) et/ou du clou centre-médullaire (R₄) constituant éventuellement la protection contre la rotation radiale.
